# EUROPÄISCHE PATENTSCHRIFT

(11) **EP 0 531 894 B1**
(45) Veröffentlichungstag und Bekanntmachung des Hinweises auf die Patenterteilung: **05.03.1997**
(21) Anmeldenummer: 92115125.4
(22) Anmeldetag: 04.09.1992
(51) Int. Cl.: G01N 33/36, B65H 63/06

(54) **Verfahren und Vorrichtung zum Klassifizieren und Reinigen von Garnen**
Method and apparatus for classifying and cleaning of yarns
Méthode et appareil pour la classification et le nettoyage des fils

(30) Priorität: 11.09.1991 CH 2673/91
(43) Veröffentlichungstag der Anmeldung: 17.03.1993
(73) Patentinhaber: ZELLWEGER LUWA AG, CH-8610 Uster (CH)
(72) Erfinder: Pidoux, Roger, CH-8047 Zürich (CH); Haldemann, Peter, CH-8832 Wollerau (CH)

(56) Entgegenhaltungen:
- EP-A- 0 005 083
- EP-A- 0 415 222
- EP-A- 0 439 767
- CH-A- 477 573
- DE-A- 1 900 312
- DE-A- 3 438 962
- DE-A- 3 928 417
- DE-A- 4 003 810

## Beschreibung

Die Erfindung betrifft ein Verfahren und eine Vorrichtung zum Klassifizieren und Reinigen von Garnen nach dem Oberbegriff der Patentansprüche 1 und 18.

Von Spindeln in Ring-, bzw. Rotorspinnmaschinen erzeugte Fäden oder Garne werden vor dem Aufspulen "gereinigt". Es werden über die Toleranzen hinausgehende Dick- und Dünnstellen, also nicht tolerierbare Fehler, herausgeschnitten und die Schnitte wieder durch Knoten oder Spleisse verbunden. Dabei werden Stellen mit grosser Abweichung vom Sollwert (= Nenndicke des Garnes) schon herausgeschnitten, wenn sie verhältnismässig kurz sind, wogegen Stellen mit geringen Abweichungen vom Sollwert nur herausgeschnitten werden, wenn sie eine grössere Länge aufweisen. Das Reinigen wird also dann eingeleitet, wenn der Garnfehler Kombinationswerte überschreitet, die einerseits aus der Abweichung von der Solldicke des Garnes und andererseits aus der Länge der Abweichung gebildet werden.

Weiter können solche Garnfehler "klassifiziert" werden. Dies bedeutet, dass man jeder Dick- oder Dünnstelle, also jedem tolerierbaren Fehler und jedem nicht tolerierbaren Fehler, einem zweidimensionalen Zahlenwert zuordnet, der im Zusammenhang sowohl zur Abweichung vom Sollwert als auch zur Länge der Abweichung steht. In der so gebildeten zweidimensionalen Zahlenebene, dem "Klassierfeld" gibt es eine irgendwie gestaltete Linie, die "Reinigungsgrenze", die festlegt, dass alle Dick- oder Dünnstellen, die ausserhalb der Reinigungsgrenze liegen und somit als nicht tolerierbare Fehler anzusehen sind, aus dem Garn herausgeschnitten werden.

Je nach der Art und Weise, in der man den zweidimensionalen Zahlenwert und die Gestalt der Reinigungsgrenze bildet, (die Wahl beider Grössen kann in verschiedener Weise geschehen,) kann man Garn mit der gleichen Dickeverteilung mit deutlich mehr oder weniger Knoten oder Spleissstellen herstellen. Je weniger Knoten oder Spleissstellen aber pro Längeneinheit des Garnes vorhanden sind und je enger die Reinigungsgrenze für die nicht tolerierbaren Fehler gewählt wurde, um so höher ist die Qualität des Garnes.

In der CH-PS 477573 wird eine Vorrichtung für einen Garnreiniger beschrieben, bei der die Garndicke in ein elektrisches Signal gewandelt, anschliessend einem Dämpfungsglied mit einstellbarer Zeitkonstante und danach einem in seiner Ansprechgrenze ebenfalls einstellbaren Schmitt-Trigger zugeführt wird, der ab einer bestimmten, eine Grösse des Garnfehlers widergebenden Impulshöhe einen Schneide- und Knüpfemechanismus betätigt. Es werden also nur Garnfehler erfasst, die von der Vorrichtung als nicht tolerierbar angesehen werden. Je nach der eingestellten Zeitkonstante werden kurze Dickstellen nahezu ungedämpft, lange, flach ansteigende Dickstellen aber deutlich gedämpft an den Schmitt-Trigger weitergegeben, was dazu führt, dass kurze Stellen nur bei erheblicher Abweichung vom Sollwert geschnitten werden, lange, flache Dickstellen aber geschnitten werden, wenn sie über eine grössere Garnlänge auch geringere Abweichung vom Sollwert als kurze Dickstellen zeigen. Gerade ein solches Verhalten ist, wie oben gesagt wurde, erwünscht. Dünnstellen können aber mit dieser Vorrichtung nicht erfasst werden.

Es ist ziemlich zeitaufwendig, diese Vorrichtung durch Änderung der Einstellwerte für die Dämpfung und für die Ansprechgrenze des Schmitt-Triggers für eine neue Garnart einzustellen. Die Vorrichtung kann ferner nicht klassifizieren, da ja nur Werte, die den Schmitt-Trigger zum Ansprechen bringen, also nicht tolerierbare Garnfehler, feststellbar sind. Es lässt sich daher keine Übersicht darüber gewinnen, wie die tolerierten Garnfehler und die geschnittenen Garnfehler verteilt liegen, so dass man keine Aussage darüber machen kann, wie weit sich die mit der Vorrichtung erreichte Reinigungsgrenze dem Optimum nähert.

Es sind ferner "Selektoren" bekannt geworden. Dies sind Karten, auf die tatsächlich aufgetretene Garnfehler zweidimensional, nach Dickenabweichung und fehlerhafter Garnlänge geordnet, aufgebracht sind. Sie werden verwendet, um für das Reinigen von Garnen die nicht tolerierbaren Fehler festzulegen. Eine Reinigungsanlage muss also so arbeiten, dass sie alle auf Grund des Selektors als nicht tolerierbar erkannten Fehler aus dem Garn ausschneidet.

Weiter wird in der EP-B1 0 153 350 ein zu Klassifizierzwecken einsetzbares Verfahren und die zugehörige Vorrichtung beschrieben, die mit ersten Prozessoren zur zyklischen Überwachung von jeweils einer grösseren Zahl von Spindeln, es wird als Beispiel die Zahl 24 angegeben, und einem zentralen Prozessor arbeitet. Die Klassifizierung des von einer Spindel gefertigten Garnes wird jeweils in einem beschränkten Überwachungszeitraum durchgeführt, dann wird das Garn der nächsten Spindel klassifiziert und so fort, bis zu einem verhältnismässig langen Zeitraum danach wieder das Garn der ursprünglichen Spindel klassifiziert wird. Die Klassifizierung wirkt also nur über einen kleinen Anteil der Laufzeit der Spindel, die Untersuchung gibt nicht alle Fehler an, sondern hat eher einen statistischen Charakter und ist nur bei einem stationären Lauf der einzelnen Spindeln brauchbar. Um die Unsicherheit einer solchen Aussage insbesondere beim Anlaufen einer Spindel zu verkleinern, wird während der Anlaufzeit einer Spindel die zyklische Untersuchung aller anderen Spindeln ausgesetzt, bis die anlaufende Spindel den stationären Zustand erreicht hat. Dies ist bei einer Spinnmaschine leicht durchführbar, da die Spindeln sukkzessive angefahren werden und daher höchstens eine Spindel sich im Anlaufzustand befindet. Dies bedeutet aber einen weiteren Ausfall an Klassifizierungszeit für die anderen Spindeln. Zum Reinigen des Garnes, bei dem alle nicht tolerierbaren Fehler erfasst werden müssen, ist diese Vorrichtung nicht brauchbar, da sie ja das von einer Spindel gefertigte Garn jeweils nur kurzzeitig auf seine Dicke untersucht.

Aus der DE-A-3928417 ist eine Steueranlage für eine Spinnmaschine bekannt, in welcher Gamfehler erfasst und angezeigt, sowie mit einem Computer besondere Arten von Gamfehlem ausgewertet werden können. Durch die Steueranlage kann auch die Reinigung und die Klassierung von Gamfehlem veranlasst werden. Dabei können aber nur solche Gamfehler klassiert werden, die vorher beim Reinigen nicht bereits herausgeschnitten wurden. Wie auch bei der aus der CH-PS-477 573 bekannten Vorrichtung, können damit Gamfehler nur entweder klassiert oder herausgeschnitten werden. Zudem spielt die Länge des Gamfehlers in dieser Steueranlage nur insofern eine Rolle, als sie erfasst werden muss weil der Gamfehler in seiner gesamten Länge erfasst und entfernt werden soll. Dagegen spielt die Länge des Gamfehlers als Kriterium für das Reinigen keine Rolle.

Der Erfindung liegt daher die Aufgabe zugrunde, ein Verfahren und eine Vorrichtung für eine mit einer Vielzahl von Spindeln arbeitenden Spinnmaschine zu schaffen, die gleichzeitig alle Garnfehler klassifiziert und das Garn jeder Spindel von nicht tolerierbaren Fehlern reinigt.

Diese Aufgabe wird durch die Kennzeichenteile der Patentansprüche 1 und 18 gelöst, die übrigen Ansprüche betreffen vorteilhafte Ausführungsmethoden.

Die Erfindung wird an Hand der Zeichnung erklärt, dabei zeigen die
- Fig. 1: das Schaltschema einer erfindungsgemässen Vorrichtung;
- Fig. 2: ein Klassierfeld;
- Fig. 3: eine gedämpfte und eine ungedämpfte Aufzeichnung von Garnfehlern; und
- Fig. 4: eine Zerlegung eines Garnfehlers in Teilfehler.

In allen Figuren der Zeichnung sind gleichartige Gegenstände mit den gleichen Beizeichen gekennzeichnet.

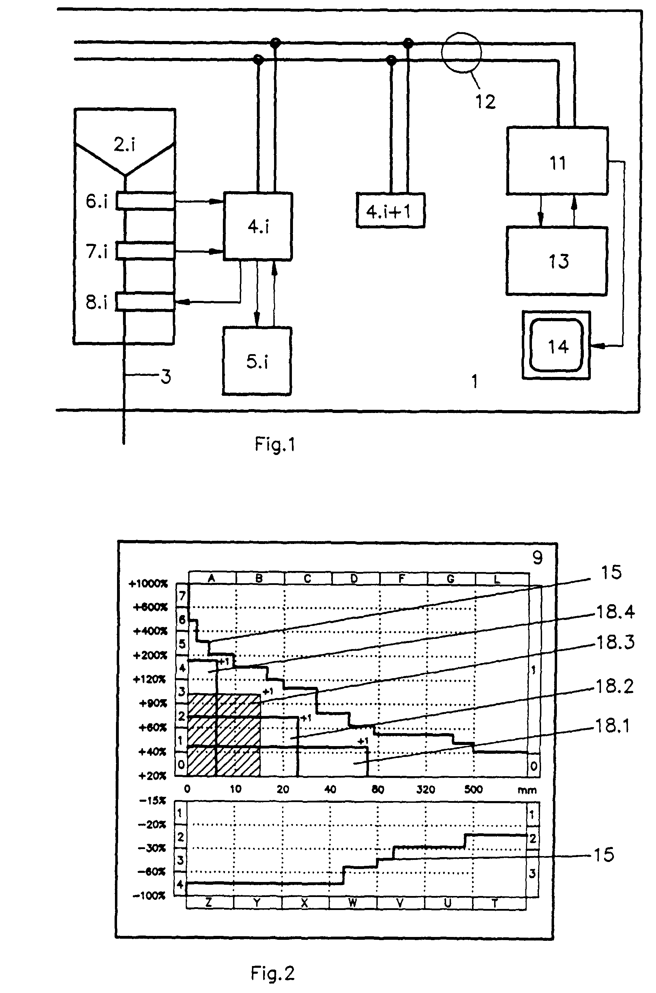

Eine erfindungsgemässe Vorrichtung ist in der Fig. 1 im Prinzip dargestellt. Sie besteht aus einer Spinn- oder Spulmaschine 1, wobei im folgenden nur Spinnmaschinen besprochen werden. Bei Spulmaschinen treten an Stelle der Spindeln jeweils (Primär-) Spulen. Jede Spindel 2.i, die ja Garn 3 herstellen, ist mit einem eigenen Spindel-Prozessor 4.i, und mit einem Spindel-Speicher 5.i ausgerüstet. Dabei empfängt der Spindel-Prozessor 4.i kontinuierlich Messwerte über die Dicke des Garnes 3 von einer Messeinrichtung für die Garndicke 6.i und für Längen des Garnes von einer Messeinrichtungen für Längen des Garnes 7.i. Der Spindel-Prozessor 4.i stellt fest, welche Garnfehler tolerierbar und welche nicht tolerierbar sind und veranlasst eine Schneideeinrichtung 8.i, die nicht tolerierbaren Garnfehler aus dem Garn 3 herauszuschneiden und das Garn 3 durch Knüpfen oder Spleissen wieder zu verbinden. Grundsätzlich ist auch die Ausrüstung mehrerer Spindeln 2.i mit je einem Spindel-Prozessor 4.i möglich, der aber dann über eine sehr viel höhere Rechenleistung verfügen muss, um die ständige Überwachung dieser Spindeln zu gewährleisten.

In der Fig. 1 ist der auf die Spindel 2.i folgende zu der nicht gezeichneten Spindel 2.i+1 gehörende nächste Spindel-Prozessor mit 4.i+1 bezeichnet.

An der Spinnmaschine befindet sich ferner ein zentraler Prozessor 11, mit dem alle Spindel-Prozessoren 4.i über einen Bus 12 verbunden sind, wobei der zentrale Prozessor 11 einen zentralen Speicher 13 und eine Anzeige 14, beispielsweise einen Bildschirm, besitzt und übergeordnete Steueraufgaben und die Ausgabe von Anzeigen durchführt.

Die Art der Auswertung der vom Spinn-Prozessor 4.i übernommenen Daten wird an Hand der Fig. 2 erklärt. Es wird ein darin dargestelltes "Klassierfeld" 9 gebildet. Der Spinn-Prozessor 4.i bestimmt für jeden Garnfehler auf Grund der Abweichung der Garndicke vom Sollwert sowie seiner fehlerbehafteten Garnlänge zweidimensionale Klassenkennzeichen. In dem dargestellten Beispiel sind für die Dickenabweichung die Zahlen von 0 bis 7 und für die fehlerbehaftete Garnlänge die Buchstaben A bis L (bei positiven Dickenabweichungen) und T bis Z (bei negativen Dickenabweichungen) verwendet. Die Dickenabweichungen werden in % angegeben, die fehlerbehafteten Garnlängen in mm. Für jedes zweidimensionale Klassenkennzeichen (also beispielsweise für A3, was im dargestellten Beispiel einer fehlerbehafteten Länge zwischen 0 und 10 mm und einer Dickenabweichung zwischen +90 und +120% entspricht), existiert im Klassierfeld 9 eine Zelle 10.i,k. Der Spinn-Prozessor vermerkt in diesen Zellen 10.i,k jeweils die Zahl der Garnfehler, für die das der Zelle 10.i,k zugehörige Klassenkennzeichen gefunden wurde. Er erhöht somit die Zahl für die bis dahin aufgelaufenen Garnfehler in einer mit dem betreffenden Klassenkennzeichen bezeichneten Zelle 10.i,k jedesmal, wenn ein Garnfehler mit diesem Klassenkennzeichen gefunden wurde. Der Garnfehler ist somit klassifiziert.

Im Klassierfeld 9 liegt eine stark ausgezogene Linie, die tolerierbare Garnfehler von den nicht tolerierbaren Garnfehlern scheidet. Dies ist die "Reinigungsgrenze" 15. Meist besteht diese Linie aus halben Rechteckstücken. Die nicht tolerierbaren Garnfehler liegen auf der Aussenseite der Reinigungsgrenze 15 und müssen aus dem Garn geschnitten werden.

Der Spindel-Prozessor 4.i kann die Zahl der Garnfehler in jeder Zelle 10.i,k auf eine vorzugebende Länge des fertigen Garnes 3 beziehen. So kann z.B. die Zahl jeweils auf 10 km Garn 3 bezogen werden.

Das Klassierfeld 9 mit den Zahlen der Garnfehler für eine vorgegebene Länge des Garnes 3 ist für jede Spindel über den zentralen Prozessor 11 an dem Bildschirm 14 anzeigbar.

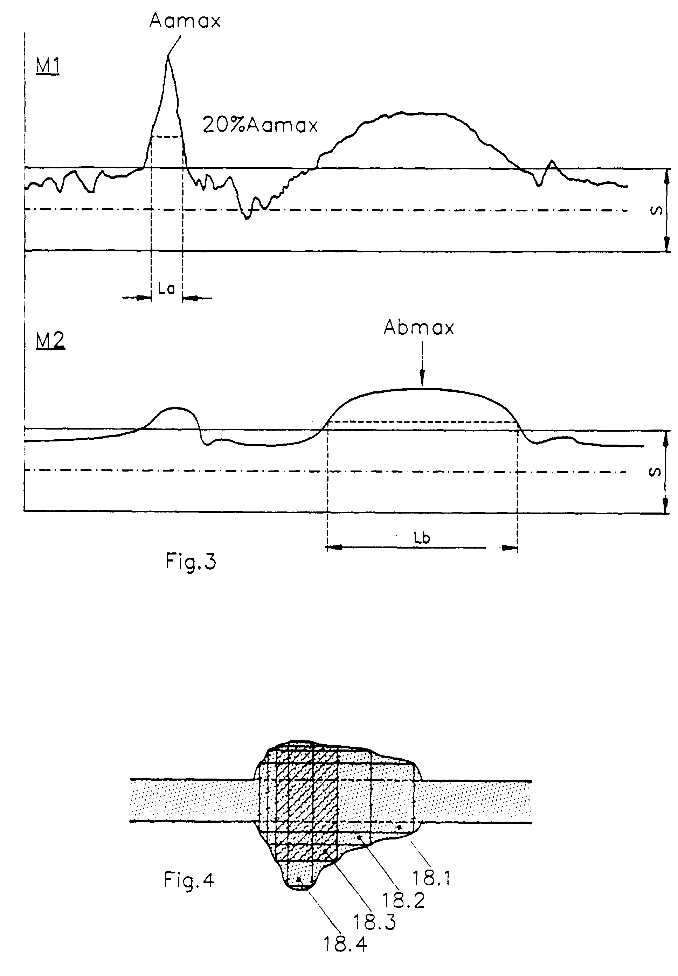

Nur in der Figur 3 werden folgende Beizeichen verwendet: die Solldicke S, die maximale Abweichung Amax von der Solldicke S und die fehlerbehaftete Garnlänge L. Mit M1 sind die ungedämpften, mit M2 deutlich gedämpfte Messwerte bezeichnet. Mit dem Index a sind diese Grössen bei einem Garnfehler mit steiler Flanke, mit dem Index b bei einem Garnfehler mit flacher Flanke gekennzeichnet.

Die Dickenmesswerte können bei Garnfehlern mit steiler Flanke als ungedämpfter Messwert M1, bei Garnfehlern mit flacher Flanke als gedämpfter Messwert M2 dem nicht gezeichneten Spindel-Prozessor 4.i zugeleitet werden. Dies führt dazu, dass bei kurzen Garnfehlern der ungedämpfte Messwert M1 gross sein muss, damit diese Garnfehler die Reinigungsgrenze 15 (Fig. 2) überschreiten.

Flache Fehler müssen aber eine grosse Fehlerlänge aufweisen, damit ihr gedämpfter Messwert M2 die Reinigungsgrenze 15 überschreitet. Dies ist ein erwünschtes Verhalten.

Für den Garnfehler verwendet der Spindel-Prozessor 4.i, wie die Fig. 3 zeigt, als fehlerbehaftete Garnlänge diejenige Länge L, bei der die Abweichung der Garndicke vom Sollwert einen vorgegebenen Bruchteil der maximalen Abweichung Amax der Garndicke vom Sollwert S bei dem betreffenden Garnfehler überschreitet. So wird beispielsweise als fehlerbehaftete Garnlänge diejenige Länge L verwendet, bei der die Abweichung der Garndicke 1/5 der maximalen Abweichung Amax der Garndicke vom Sollwert bei dem betreffenden Garnfehler überschreitet. Es können aber auch andere Werte verwendet werden.

Ein Garnfehler kann beispielsweise, wie die Fig. 4 zeigt, in Teilfehler 18 aufgeteilt werden, welche als zur Garnachse parallele, kreisförmige, eingezeichnete Prismen in der zur Garnachse parallelen Schnittfläche des Garnfehlers darstellbar sind. Für jeden Teilfehler 18 lässt sich dann auf Grund seiner Prismenlänge und der Abweichung seines Prismenradius von dem Sollwert der Garndicke ein Klassenkennzeichen berechnen. Dies ist mit den Teilfehlern 18.1 bis 18.4 nach der Fig. 4 durchgeführt, diese Teilfehler 18.1 bis 18.4 sind in der Fig. 2 eingetragen. In der zugehörigen Zelle 10.i,k wird dann die Zahl der Fehler um eins erhöht. Ein Garnfehler gilt in diesem Falle als nicht tolerierbar und sein Herausschneiden muss vom Spindel-Prozessor 4.i durchgeführt werden, wenn mindestens einer seiner Teilfehler 18 ein Klassenkennzeichen ausserhalb der Reinigungsgrenze 15 aufweist. Dies ist bei den Teilfehlern 18.1 bis 18.4 in dem Klassierfeld 9 mit der dort eingezeichneten Reinigungsgrenze 15 nicht der Fall. Die hier beschriebene Aufzeichnungsweise von Teilfehlern 18 ergibt eine feiner unterteilte Klassifizierung.

Bei einem durch Eintrag von genügend grossen Zahlen von Garnfehlern in die Zellen 10.i,k gesichertem Klassierfeld 9 kann die Reinigungsgrenze 15 dadurch verbesserbar sein, dass sie in der Umgebung von Zellen 10.i,k mit hohen Zahlen von Garnfehlern nach aussen, in der Umgebung von Zellen 10.i,k mit niederen Zahlen von Garnfehlern nach innen verlegt wird. Auf diese Weise wird die Zahl der notwendigen Knoten oder Spleisse verringert, bei gleicher Menge Ausgangsmaterial die Länge des fertigstellbaren Garnes vergrössert.

Die erfindungsgemässe Vorrichtung und das erfindungsgemässe Verfahren ermöglichen so, alle an einer Spinnmaschine 1 von vielen Spindeln 2.i gleichzeitig gefertigte Garne 3 auch gleichzeitig zu reinigen und zu klassifizieren. Dadurch ist die gestellte Aufgabe gelöst.

Die Garnfehler werden in ein Klassierfeld 9 eingetragen, in dem eine Reinigungsgrenze 15 liegt, mit deren Hilfe die nicht tolerierbaren Fehler bestimmt werden. Die Einrichtung ermöglicht es, nach einer gewissen Anlaufzeit der Spindel 2.i, diese Reinigungsgrenze 15 so zu verändern, dass die Zahl der Knoten oder Spleisse im Garn 3 bei Einhaltung der Fertigungsbedingungen für das Garn 3 zu einem Minimum wird, wodurch die Qualität und die gefertigte Garnlänge beim Einsatz der gleichen Materialmenge erhöht wird.

## Patentansprüche

1. Verfahren zum Klassifizieren und Reinigen von Garnen (3) an einer Spinn- oder Spulmaschine (1) mit einer Vielzahl von Spindeln, die Garn mit Fehlern verschiedener Dicke und Länge verarbeiten, bei dem kontinuierlich Messwerte über die Dicke und die Länge des Garns von jeder Spindel (2.i) einem Spindel-Prozessor (4.i) zugeführt werden und bei dem ein zweidimensionales Klassierfeld (9) mit Zellen (10) zur Registrierung von Garnfehlern verwendet wird, dadurch gekennzeichnet, dass
auf Grund von Abweichungen der Messwerte (A) von einem Sollwert (S) der Garndicke sowie der fehlerbehafteten Garnlänge (L) ein Garnfehler erkannt und davon zweidimensionale Klassenkennzeichen bestimmt werden, dass
eine Zahl für die bis dahin aufgelaufenen Garnfehler in einer mit diesen Klassenkennzeichen bezeichneten Zelle (10.i,k) des zweidimensionalen Klassierfeldes (9) erhöht und anschliessend veranlasst wird, dass
der Garnfehler, sofern er im zweidimensionalen Klassierfeld (9) einer, ausserhalb einer Reinigungsgrenze (15) liegenden Zelle zugeordnet wird, aus dem Garn herausgeschnitten wird.

2. Verfahren nach Anspruch 1 an einer Spulmaschine, wobei das in diesen Patentansprüchen für Spindeln (2.i) gesagte für die Primärspulen gilt.

3. Verfahren nach Anspruch 1 oder 2, dadurch gekennzeichnet, dass der Spindel-Prozessor (4.i) die Zahl der Garnfehler in jeder Zelle (10.i,k) auf eine vorgegebene Länge des zu fertigenden Garnes (3) bezieht.

4. Verfahren nach Anspruch 1 oder 2, dadurch gekennzeichnet, dass für jede Spindel (2.i) das Klassierfeld (9) mit den Zahlen der Garnfehler über den zentralen Prozessor (11) an einem Bildschirm (14) anzeigbar ist.

5. Verfahren nach Anspruch 1 oder 2, dadurch gekennzeichnet, dass dem Spindel-Prozessor (4.i) ungedämpfte Messwerte (M1) und gedämpfte Messwerte (M2) für die Abweichung (A) der Garndicke vom Sollwert (S) zugeführt werden.

6. Verfahren nach Anspruch 5, dadurch gekennzeichnet, dass für Garnfehler mit steiler Flanke die ungedämpften Messwerte (M1) dem Spindel-Prozessor (4.i) zugeführt werden.

7. Verfahren nach Anspruch 5, dadurch gekennzeichnet, dass für Garnfehler mit flacher Flanke die gedämpften Messwerte (M2) dem Spindel-Prozessor (4.i) zugeführt werden.

8. Verfahren nach Anspruch 1 oder 2, dadurch gekennzeichnet, dass der Spindel-Prozessor (4.i) für den Garnfehler als fehlerbehaftete Garnlänge (L) diejenige Länge verwendet, bei der die Abweichung (A) der Garndicke vom Sollwert (S) einen vorgegebenen Bruchteil der maximalen Abweichung (A) der Garndicke vom Sollwert (s) bei dem betreffenden Garnfehler überschreitet.

9. Verfahren nach Anspruch 8, dadurch gekennzeichnet, dass für den Garnfehler als fehlerbehaftete Garnlänge (L) diejenige Länge verwendet wird, bei der die Abweichung (A) der Garndicke vom Sollwert (S) 1/5 der maximalen Abweichung (Amax) der Garndicke vom Sollwert (A) bei dem betreffenden Garnfehler überschreitet.

10. Verfahren nach Anspruch 1 oder 2, dadurch gekennzeichnet, dass für jeden Garnfehler ein Klassenkennzeichen berechnet wird und in der zugehörigen Zelle (10.i,k) die Zahl der Fehler um eins erhöht wird.

11. Verfahren nach Anspruch 10, dadurch gekennzeichnet, dass ein Garnfehler als nicht tolerierbar gilt und sein Herausschneiden durch den Spindel-Prozessor (4.i) zu veranlassen ist, wenn er ein Klassenkennzeichen ausserhalb der Reinigungsgrenze (15) aufweist.

12. Verfahren nach Anspruch 1 oder 2, dadurch gekennzeichnet, dass ein Garnfehler in Teilfehler (18.i) aufgeteilt wird.

13. Verfahren nach Anspruch 12, dadurch gekennzeichnet, dass die Teilfehler (18.i) als der Garnachse parallele, kreisförmige Innen-Prismen in die der Garnachse parallele Schnittfläche des Garnfehlers einzeichenbar sind.

14. Verfahren nach Anspruch 12, dadurch gekennzeichnet, dass für jeden Teilfehler (18.i) ein Klassenkennzeichen berechnet wird und in der zugehörigen Zelle (10.i,k) die Zahl der Fehler um eins erhöht wird.

15. Verfahren nach Anspruch 14, dadurch gekennzeichnet, dass ein Fehler als nicht tolerierbar gilt und sein Herausschneiden vom Spindel-Prozessor (4.i) zu veranlassen ist, wenn mindestens einer seiner Teilfehler (18.i) ein Klassenkennzeichen ausserhalb der Reinigungsgrenze (15) aufweist.

16. Verfahren nach Anspruch 1 oder 2, dadurch gekennzeichnet, dass bei einem durch Eintrag von genügend hohen Zahlen von Garnfehlern in die Zellen (10.i,k) gesichertem Klassierfeld (9) die Reinigungsgrenze (15) dadurch verbesserbar ist, dass sie in der Umgebung von Zellen (10.i,k) mit hohen Zahlen von Garnfehlern nach aussen, in der Umgebung von Zellen (10.i,k) mit niederen Zahlen von Garnfehlern nach innen verlegt wird.

17. Verfahren nach einem der Ansprüche 1 bis 16, dadurch gekennzeichnet, dass ein Spindel-Prozessor (4.i) für mehrere Spindeln (2.i) arbeitet.

18. Vorrichtung zur Durchführung des Verfahrens nach einem der Ansprüche 1 bis 17, mit einem zentralen Prozessor (11) mit einem zentralen Speicher (13) und einem Bildschirm (14) und für jede Spindel (2.i) einen Spindel-Prozessor (4.i) mit einem Spindel-Speicher (5.i), wobei die Spindel-Prozessoren (4.i) mit einer Messeinrichtung für die Gamdicke (6.i) des von der zugehörigen Spindel (2.i) gefertigten Games (3) verbunden sind, von den Spindel-Prozessoren (4.i) ein Klassierfeld (9) erzeugbar ist, das Zellen enthält, die durch zweidimensionale Klassenkennzeichen gekennzeichnet sind, die einerseits durch eine Abweichung (A) von einem Sollwert (S) der Gamdicke, andererseits von der fehlerbehafteten Gamlänge (L) von Gamfehlem bestimmbar sind und durch den Spindel-Prozessor (4.i) in den Zellen des Klassierfeldes (9) Zahlen über die Anzahl Gamfehler eintragbar sind, dadurch gekennzeichnet, dass vom Spindel-Prozessor (4.i) in das Klassierfeld eine Reinigungsgrenze (15) legbar ist, durch den Spindel-Prozessor (4.i) das Herausschneiden der ausserhalb der Reinigungsgrenze (15) liegenden Gamfehler ausgehend von der Klassierung des Gamfehlers veranlassbar ist und über den zentralen Prozessor (11) und den Bildschirm (14) das Klassierfeld (9) für jede Spindel (2.i) anzeigbar ist.

## Claims

1. Method for the classifying and cleaning of yarns (3) on a spinning or winding machine (1) with several spindles which process yarn with faults of differing thicknesses and lengths, in which measurement values relating to the thickness and the length of the yarn from each spindle (2.i) are supplied continuously to a spindle processor (4.i) and in which a two dimensional classifying table (9) with cells (10) is used for registering yarn faults, characterized in that
a yarn fault is detected on the basis of deviations (A) of the measurement values from a required value (S) of the yarn thickness and of the faulty yarn length (L) and from this is determined a two-dimensional class identification, in that
a number for the yarn faults accumulated to that point is incremented in a cell (10.i,k) of the two-dimensional classifying table (9) designated with this class identification and is then activated, and in that
the yarn fault is cut out from the yarn if it is assigned to a cell located outside a cleaning limit (15) in the two-dimensional classifying table (9).

2. Method according to Claim 1 on a winding machine, whereby that which is stated in these Claims for spindles (2.i) applies to the primary bobbins.

3. Method according to Claim 1 or 2, characterized in that the spindle processor (4.i) relates the number of the yarn faults in each cell (10.i,k) to a predefined length of the yarn (3) to be produced.

4. Method according to Claim 1 or 2, characterized in that the classifying table (9) with the numbers of the yarn faults can be displayed on the screen (14) by the central processor (11) for each spindle (2.i).

5. Method according to Claim 1 or 2, characterized in that non-attenuated measurement values (M1) and attenuated measurement values (M2) for the deviation (A) of the yarn thickness from the required value (S) are supplied to the spindle processor (4.i).

6. Method according to Claim 5, characterized in that, for yarn faults with a steep profile, the non-attenuated measurement values (M1) are supplied to the spindle processor (4.i).

7. Method according to Claim 5, characterized in that, for yarn faults with a flat profile, the attenuated measurement values (M2) are supplied to the spindle processor (4.i).

8. Method according to Claim 1 or 2, characterized in that the faulty yarn length (L) used by the spindle processor (4.i) for the yarn fault is the length, in which the deviation (A) of the yarn thickness from the required value (S) exceeds a predefined fraction of the maximum deviation (A) of the yarn thickness from the required value (S) for the yarn fault concerned.

9. Method according to Claim 8, characterized in that the faulty yarn length (L) used for the yarn fault is the length in which the deviation (A) of the yarn thickness from the required value (S) exceeds 1/5 of the maximum deviation (Amax) of the yarn thickness from the required value (A) for the yarn fault concerned.

10. Method according to Claim 1 or 2, characterized in that a class identification is calculated for each yarn fault and the number of faults is incremented by one in the associated cell (10.i,k).

11. Method according to Claim 10, characterized in that a yarn fault is considered non-acceptable and its cutting out is to be initiated by the spindle processor (4.i) if it has a class identification outside the cleaning limit (15).

12. Method according to Claim 1 or 2, characterized in that a yarn fault is divided into sub-faults (18.i).

13. Method according to Claim 12, characterized in that the sub-faults (18.i) can be drawn into the section of the yarn fault parallel to the yarn axis as circular inner prisms parallel to the yarn axis.

14. Method according to Claim 12, characterized in that a class identification is calculated for each sub-fault (18.i) and the number of faults is incremented by one in the associated cell (10.i,k).

15. Method according to Claim 14, characterized in that a fault is considered non-acceptable and its cutting out is to be initiated by the spindle processor (4.i) if at least one of its sub-faults (18.i) has a class identification outside the cleaning limit (15).

16. Method according to Claim 1 or 2, characterized in that if a classifying table (9)is rendered reliable by the entry of sufficiently large numbers of yarn faults in the cells (10.i,k), the cleaning limit (15) can be improved by moving it outwards in the area around cells (10.i,k) with high numbers of yarn faults and inwards in the area around cells (10.i,k) with low numbers of yarn faults.

17. Method according to any one of Claims 1 to 16, characterized in that one spindle processor (4.i) operates for several spindles (2.i).

18. Device for the execution of the method according to any one of Claims 1 to 17, with a central processor (11) with a central memory (13) and a monitor screen (14) and, for each spindle (2.i), a spindle processor (4.i) with a spindle memory (5.i), the spindle processors (4.i) being connected to a measuring device (6.i) for measuring the yarn thickness of the yarn (3) produced from the associated spindle (2.i), the spindle processors (4.i) being capable of generating a classifying table (9) containing cells which are identified by two-dimensional class identifications which can be determined through both a deviation (A) of the yarn thickness from the required value (S) and the faulty yarn length (L) of yarn faults and the spindle processors (4.i) being capable of entering numbers relating to the quantity of yarn faults into the cells of the classifying table (9), characterized in that the spindle processor (4.i) can enter a cleaning limit (15) in the classifying table, the spindle processor (4.i) can initiate the cutting out of the yarn faults located outside the cleaning limit (15) on the basis of the classification of the yarn fault and the classifying table (9) can be displayed for each spindle (2.i) through the central processor (11) and the monitor screen (14).

## Revendications

1. Procédé de classification et de nettoyage de fils (3) sur une machine à filer et à bobiner (1), avec une pluralité de broches, travaillant un fil manifestant des erreurs, de différentes épaisseur et longueur, pour laquelle on fournit à un processeur de broche (4.i) en continu des valeurs de mesure concernant l'épaisseur et la longueur du fil en provenance de chaque broche (2.i) et pour laquelle on utilise un champ de classification (9) bidimensionnel avec des cellules (10) pour l'enregistrement des erreurs de fil, caractérisé en ce que
sur la base des écarts entre les valeurs de mesure (A) et une valeur de consigne (S) de l'épaisseur de fil, ainsi que de la longueur de fil (L) entachée d'erreurs, on identifie une erreur de fil et l'on détermine à partir de cela des caractéristiques de classe bidimensionnelles, en ce que
un nombre de fois pour lequel des erreurs de fil s'étant produites jusqu'ici dans une cellule (10.i, k) désignée par cette caractéristique de classe, du champ de classification (9) bidimensionnel est augmenté, et qu'il est pris soin ensuite que
l'erreur de fil, dans la mesure où elle est associée, dans le champ de classification bidimensionnel (9), à une cellule, située hors d'une limite de nettoyage (15), soit éliminée du fil par découpage.

2. Procédé selon la revendication 1 sur une machine à bobiner, les indications données pour les broches (2.i) dans ces revendications de brevet valant pour les bobines primaires.

3. Procédé selon la revendication 1 ou 2, caractérisé en ce que le processeur de broche (4.i) relie le nombre d'erreurs de fil dans chaque cellule (10.i, k) à une longueur prédéterminée du fil (3) à fabriquer.

4. Procédé selon la revendication 1 ou 2, caractérisé en ce que, pour chaque broche (2.i), le champ de classification (9) peut être indiqué par les nombres d'erreurs de fil, par l'intermédiaire du processeur central (11), sur un écran d'affichage (14).

5. Procédé selon la revendication 1 ou 2, caractérisé en ce que l'on amène au processeur de broche (4.i) des valeurs de mesure (M1) non amorties et des valeurs de mesure (M2) amorties valant pour l'écart (A) entre l'épaisseur de fil et la valeur de consigne (S).

6. Procédé selon la revendication 5, caractérisé en ce que, pour des erreurs de fil pour lesquelles on a des flancs raides, les valeurs de mesure (M1) non amorties sont amenées au processeur de broche (4.i).

7. Procédé selon la revendication 5, caractérisé en ce que l'on amène, pour des erreurs de fil pour lesquelles on a des flancs flats, les valeurs de mesure (M2) amorties au processeur de broche (4.i).

8. Procédé selon la revendication 1 ou la revendication 2, caractérisé en ce que le processeur de broche (4.i) affecté à l'erreur de fil utilise, comme longueur de fil (L) entachée d'erreurs, la longueur pour laquelle l'écart (A) entre l'épaisseur de fil et la valeur de consigne (S) dépasse une fraction prédéterminée de l'écart maximal (A) de l'épaisseur de fil vis-à-vis de la valeur de consigne (S) pour l'erreur de fil concernée.

9. Procédé selon la revendication 8, caractérisé en ce que, pour l'erreur de fil'exprimée sous forme de longueur de fil (L) entachée d'erreurs, on utilise la longueur pour laquelle l'écart (A) entre l'épaisseur de fil et la valeur de consigne (S) dépasse 1/5 de l'écart maximal (Amax) entre l'épaisseur de fil et la valeur de consigne (A) pour l'erreur de fil concernée.

10. Procédé selon la revendication 1 ou 2, caractérisé en ce que, pour chaque erreur de fil, on calcule une caractéristique de classe et, dans la cellule (10.i, k) afférente, on augmente de la valeur un le nombre des erreurs.

11. Procédé selon la revendication 10, caractérisé en ce qu'une erreur de fil est considérée comme non tolérable et son élimination par coupure, par le processeur de broche (4.i), étant autorisée lorsqu'il présente une caractéristique de classe se trouvant hors de la limite de nettoyage (15).

12. Procédé selon la revendication 1 ou 2, caractérisé en ce que l'on découpe une erreur de fil en erreurs partielles (18.i).

13. Procédé selon la revendication 12, caractérisé en ce que les erreurs partielles (18.i) peuvent être mentionnées sous la forme de prismes intérieurs à forme de cercle parallèles à l'axe de fil, dans la surface de coupe parallèle à l'axe de fil de l'erreur de fil.

14. Procédé selon la revendication 12, caractérisé en ce que l'on calcule pour chaque erreur partielle (18.i) une caractéristique de classe et l'on augmente le nombre des erreurs de un dans la cellule (10.i, k) afférente.

15. Procédé selon la revendication 14, caractérisé en ce qu'une erreur est considérée comme non tolérable et son élimination par coupure par le processeur de broche (4.i) étant autorisée lorsqu'au moins l'une de ses erreurs partielles (18.i) présente une caractéristique de classe hors de la limite de nettoyage (15).

16. Procédé selon la revendication 1 ou 2, caractérisé en ce que, dans le cas d'un champ de classification (9) assuré par l'introduction de nombres suffisamment élevés d'erreurs de fil dans les cellules (10.i, k), la limite de nettoyage (15) est susceptible d'être améliorée par le fait qu'elle est déplacée vers l'extérieur dans l'environnement de cellules (10.i, k) ayant de grands nombres d'erreurs de fil, et qu'elle est déplacée vers l'intérieur dans l'environnement de cellules (10.i, k) ayant de moindres nombres d'erreurs de fil.

17. Procédé selon l'une des revendications 1 à 16, caractérisé en ce qu'un processeur de broche (4.i) travaille pour plusieurs broches (2.i).

18. Dispositif de mise en oeuvre du procédé selon l'une des revendications 1 à 17, avec un processeur central (11) équipé d'une mémoire centrale (13) et d'un écran d'affichage (14) et, pour chaque broche (2.i), un processeur de broche (4.i) ayant une mémoire de broche (5.i), les processeurs de broche (4.i) étant reliés à un dispositif de mesure destiné à appréhender l'épaisseur de fil (6.i) du fil (3) fabriqué par la broche (2.i) afférente, un champ de classification (9) étant susceptible d'être généré par les processeurs de broche (4.i) et contenant des cellules qui sont caractérisées par des caractéristiques de classe bidimensionnelles qui sont susceptibles d'être déterminées, d'une part, par un écart (A) vis-à-vis d'une valeur de consigne (S) de l'épaisseur de fil, d'autre part, par la longueur de fil (L) entachée d'erreurs, et des nombres concernant le nombre d'erreurs de fil pouvant être introduits par le processeur de broche (4.i) dans les cellules du champ de classification (9), caractérisé en ce qu'une limite de nettoyage (15) peut être placée par le processeur de broche (4.i) dans le champ de classification, l'enlèvement par découpage des erreurs de fil situées hors de la limite de nettoyage (15), en partant de la classification de l'erreur de fil pouvant être autorisé par le processeur de broche (4.i), et le champ de classification (9) de chaque broche (2.i) étant susceptible d'être affiché par l'intermédiaire du processeur central (11) et de l'écran (14).
